# EUROPÄISCHE PATENTSCHRIFT

(11) **EP 0 869 807 B1**
(45) Veröffentlichungstag und Bekanntmachung des Hinweises auf die Patenterteilung: **05.03.2003**
(21) Anmeldenummer: 96946094.8
(22) Anmeldetag: 18.12.1996
(51) Int. Cl.: A61K 35/78, A61P 31/12

(54) **ANWENDUNG EINES MEDIKAMENTS UND VERWENDUNG EINES STOFFGEMISCHS ZUR HERSTELLUNG EINES MEDIKAMENTS**
USE OF A MEDICAMENT AND USE OF MIXTURE OF SUBSTANCES TO PRODUCE A MEDICAMENT
EMPLOI D'UN MEDICAMENT ET UTILISATION D'UN MELANGE DE SUBSTANCES POUR LA PRODUCTION D'UN MEDICAMENT

(30) Priorität: 18.12.1995 DE 19547317
(43) Veröffentlichungstag der Anmeldung: 14.10.1998
(73) Patentinhaber: Dreluso Pharmazeutika Dr. Elten & Sohn GmbH, 31840 Hessisch Oldendorf (DE)
(72) Erfinder: ELTEN, Holger, D-31840 Hessisch Oldendorf (DE); STEINBECK-KLOSE, Annemarie, D-53129 Bonn (DE)
(74) Vertreter: Seewald, Jürgen, Dipl.-Ing.
(86) Internationale Anmeldenummer: DE9602444
(87) Internationale Veröffentlichungsnummer: WO97022354

(56) Entgegenhaltungen:
- EP-A- 0 599 307
- WO-A-89/01329
- WO-A-89/09056
- BIOCHEMICAL AND BIOPHYSICAL RESEARCH COMMUNICATIONS, Bd. 172, Nr. 1, 15.Oktober 1990, ORLANDO, FL US, Seiten 149-153, XP002035683 GEORGE A. KRAUS ET AL.: "ANTIRETROVIRAL ACTIVITY OF SYNTHETIC HYPERICIN AND RELATED ANALOGS"
- PROCEEDINGS OF THE NATIONAL ACADEMY OF SCIENCES OF USA, Bd. 85, Nr. 14, 1.Juli 1988, Seiten 5230-5234, XP000569607 MERUELO D ET AL: "THERAPEUTIC AGENTS WITH DRAMATIC ANTIRETROVIRAL ACTIVITY AND LITTLE TOXICITY AT EFFECTIVE DOSES: AROMATIC POLYCYCLIC DIONES HYPERICIN AND PSEUDOHYPERICIN"

## Beschreibung

Die Erfindung betrifft die Verwendung eines Stoffgemischs auf der Basis von Wirkstoffen der Pflanze Hypericum (Johanniskraut) zur Herstellung eines Medikaments.

Die Pflanze Johanniskraut sowie deren Verwendbarkeit als Arznei waren bereits den Völkern der Antike bekannt. Die meisten der weltweit ca. 370 Arten dieser Pflanze enthalten den roten Farbstoff Hypericin, ein Naphtodianthronderivat mit einem Molekulargewicht von 504,43 und der Summenformel C₃₀H₁₆O₈. Neben Hypericin wurde eine Reihe von Strukturanaloga identifiziert, die in der Literatur als Pseudohypericin, Protohypericin, Protopseudohypericin, Cyclopseudohypericin, Isohypericin, Kielcortin etc.-sogenannte polycyklische Dionen - beschrieben wurden. Andere In-haltsstoffe der Pflanze sind z.B. das Flavonoid Quercetin sowie andere Bioflavonoide und Flavonoid-Glykoside, Pflanzensäuren wie Chlorogen- und Kaffeesäure, Hyperforin - ein Phloroglucinderivat - Gerbstoffe, Blütenfarbstoffe wie Cyanidinchlorid und Xantophyll, Anthrachinone, Xantonderivate, etherische Öle wie Terpineol, harzartige Stoffe, Fette und Wachse.

Neuerdings ist bekannt geworden, daß polycyklische Dione (Hypericin, Pseudohypericin und/oder Salze davon) in hochreiner Form eine antivirale Wirksamkeit besitzen. Insbesondere konnte in vitro eine hemmende Wirkung auf Retroviren beobachtet werden (vgl. EP 0 332 697 B1; dies gilt auch in bezug auf das humane Immunodefizienz-Virus (HIV), welches beim Menschen die erworbene Immunschwächekrankheit (AIDS) hervorruft. Hierbei wurde Hypericin oder Pseudohypericin in hochreiner Form verwendet, für dessen Gewinnung das Extrakt aus der Pflanze Hypericum so lange behandelt wurde, bis nahezu reines Hypericin/Pseudohypericin zur Verfügung stand. Alternativ wurde hochreines Hypericin synthetisch hergestellt. Die in diesem Zusammenhang vorgeschlagenen Medikamente enthielten nahezu reines Hypericin/Pseudohypericin (oder pharmazeutisch wirksame Salze davon) als einzigen aktiven Wirkstoff, und dazu in bekannter Weise für verschiedene Darreichungsformen und Dosierungen geeignete pharmakologisch bekannte Lösemittel und Trägersubstanzen.

Auch ist vorgeschlagen worden, zur Behandlung von durch Retroviren verursachten Krankheiten die Gabe von Hypericin/Pseudohypericin mit der Gabe von Nukleosidanaloga zu kombinieren (vgl PCT/US89/01211). Auch hierbei wurde jedoch nahezu reines Hypericin/Pseudohypericin verwendet.

Allerdings hat sich im Tierversuch erwiesen, daß bei Anwendung von hochreinem Hypericin/Pseudohypericin starke Nebenwirkungen in Form von Hyper-Photosensibilisierungen auftreten können. Aufgrund dieses Effektes verbietet sich eine therapeutische Anwendung von Präparaten mit hochreinem Hypericin/Pseudohypericin. Dieser Ansatz wurde daher nicht weiterverfolgt, und es kamen keine Hypericum-Präparate zur antiviralen Anwendung auf den Harkt.

In der Medizin werden daneben seit langem Hypericum-Präparate auf der Basis von Extrakten der Pflanze für verschiedene Indikationen im psychogenen Bereich wie Depressionen ohne endogene Formen, Schlafstörungen und psychogen bedingte sonstige Störungen wie etwa Bettnässen eingesetzt. Daneben ist eine ölige Zubereitung aus der Pflanze (Oleum Hyperici) zur externen Anwendung in der Wund- und Schmerzbehandlung bekannt. Intern wird Oleum Hyperici bei dyspeptischen Beschwerden empfohlen.

Die derzeit gültige Monographie der Kommission E beim Bundesamt für Arzneimittel und Medizinprodukte nennt als Anwendungsgebiete psychovegetative Störungen, depressive Verstimmungszustände, Angst und/oder nervöse Unruhe. Alle derzeit bekannten Hypericum-haltigen Arzneimittel betreffen diesen Indikationsbereich psychogene Störungen und Schlafstörungen.

Die bekannten Hypericum-haltigen Arzneimittel basieren auf dem Extrakt aus der Pflanze mit einem Gehalt an polycyklischen Dionen (Hypericin, Pseudohypericin und/oder Salzen davon) von weniger als 1%. Die genannten Wirkstoffe liegen also in einem nicht reinen Zustand vor. Der Einsatz derartiger Präparate für den Indikationsbereich Viruserkrankungen ist nicht bekannt.

Dieser Indikationsbereich war bislang nur vorgeschlagen für hochreine Hypericin/Pseudohypericin-Präparate, die aber aufgrund der oben genannten Nebenwirkungen therapeutisch nicht zum Einsatz kommen können.

Aufgabe der Erfindung ist es daher, ein Stoffgemisch zur Herstellung eines Medikaments vorzuschlagen, welches die antivirale, insbesondere antiretrovirale Wirkung von polycyklischen

Dionen (Hypericin, Pseudohypericin und/oder Salzen davon) unter weitgehender Vermeidung der beschriebenen Nebenwirkungen zur Behandlung einer durch einen Virus verursachten Krankheit nutzbar macht.

Diese Aufgabe wird durch die in den Ansprüchen 1 und 2 angegebene Erfindung gelöst.

Vorteilhafte Ausgestaltungen der Erfindung ergeben sich aus den Unteransprüchen.

Die Erfindung beruht auf der überraschenden Erkenntnis, daß die für hochreines Hypericin/pseudohypericin bekannte Hemmung von Retroviren auch mit Hypericin/Pseudohypericin in niedrigerer Konzentration erreicht werden kann, sofern ausreichende Dosierungen von Hypericin/Pseudohypericin verabreicht werden. Hierfür wird der Wirkstoff (Hypericin, Pseudohypericin und/oder Salze davon) mit anderen, als Bestandteile der Pflanze Hypericum bekannten Stoffen vermischt oder es werden Extrakte verwendet, die den Wirkstoff (Hypericin, Pseudohypericin und/oder Salze davon) in ähnlich geringer oder leicht konzentrierterer Form als bei den zur Behandlung von psychogenen Störungen und Schlafstörungen bekannten Präparaten, also in Konzentrationen von zwischen 0,05% und 50% an polycyklischen Dionen, jedoch nicht in hochreiner Form enthalten.

Hierbei treten überraschenderweise die im Zusammenhang mit hochreinem Hypericin/Pseudohypericin bekannten Nebenwirkungen nicht auf, und zwar auch bei Dosierungen, die bisher für Hypericumextraktpräparate für andere Indikationen (psychogene Störungen, Schlafstörungen) verwendete Dosierungen um ein Vielfaches übertreffen. Das aus der Pflanze gewonnene Extrakt, mit Konzentrationen an polycyklischen Dionen zwischen 0,05% und 50% unter Beibehaltung anderer Inhaltsstoffe der Pflanze kann demnach therapeutisch zur Behandlung von Viruserkrankungen eingesetzt werden.

Bei einer bevorzugten Ausführungsform erfolgt die Herstellung des Trockenextraktes aus der Pflanze Hypericum perforatum Linne nach den Regeln des Deutschen Arzneibuches (DAB) unter Verwendung von Ethanol 60% V/V. Die getrocknete und geschnittene Droge wird im Verhältnis von 1:4 (Droge zu Extraktionsmittel) mit Ethanol vermischt und mindestens 14 Tage unter gelegentlichem Umrühren oder Schütteln stehen gelassen. Danach wird dekantiert und der Rückstand abgepreßt.

Die Anreicherung der polycyklischen Dione (Hypericin und verwandter Stoffe) wird erreicht durch Abtrennung hochmolekularer Extraktbestandteile mittels präparativer Gelchromatographie (Molekularsiebe). Verwendet werden-Gele vom Typ Sephadex® G200.

Dem nach obigem Verfahren erlangten flüssigen Extrakt wird behutsam im Vakuum Ethanol, Wasser und flüchtige Bestandteile entzogen und zwar so lange, bis eine dickflüssige bräunliche Flüssigkeit entsteht. Diese wird auf eine präparative Chromatographiesäule aufgetragen und im Verlauf des Chromatographie-Verfahrens in Fraktionen mit aufsteigendem Molekulargewicht aufgetrennt. Die höchstmolekularen Stoffe erscheinen als letzte am Ende der Säule und werden verworfen.

Durch Optimierung der Elutionsbedingungen an der Säule in Verbindung mit laufenden Gehaltsbestimmungen wird durch die oben beschriebene Fraktionierung - durch geeignete Auswahl der gewünschten Fraktionen und verwerfen unerwünschter Fraktionen - der Gehalt des späteren Extraktes an Hypericin/Pseudohypericin sehr genau auf 1% g/g bestimmt,

Die gewünschten Fraktionen werden gesammelt und behutsam im Vakuum getrocknet. Das dabei entstehene bräunliche Pulver mit einem Gehalt an Hypericin/Pseudohypericin von 1% g/g wird direkt zur Herstellung von festen oralen Zubereitungen verwendet.

In einer weiteren bevorzugten Ausführungsform ist die feste orale Darreichungsform mit einem magensaft-resistenten Überzug versehen. Die dadurch im Duodenum auftretende Resorption erlaubt die Reduzierung der Dosis.

Orale Zubereitungen wie z.B. Kapseln oder Tabletten mit Dosierungen des oben beschriebenen Extrakts entsprechend einer Menge von 2 mg je Dosis, verabreicht in Tagesdosen von 6 mg und mehr über einen längeren Zeitraum (mindestens 6 Monate) sind in der Lage Retroviren im Serum unter die Nachweisgrenze zu bringen.

Damit kann der Ausbruch von Krankheiten, die durch Retroviren verursacht weren, wie z.B. AIDS, verhindert werden.

## Patentansprüche

1. Verwendung eines Stoffgemischs, bestehend aus einem oder mehreren polycyklischen Dionen, gewählt aus Hypericin (C₃₀H₁₆O₈), Pseudohypericin und Salzen davon, in Konzentration zwischen 0,05% und 50%, sowie aus einem oder mehreren weiteren in der Pflanze Hypericum (Johanniskraut) vorkommenden Inhaltsstoffen in Konzentration zwischen 50% und 99,95% zur Herstellung eines Medikaments zur Behandlung einer Krankeit, verursacht durch einen Virus.

2. Verwendung eines Stoffgemischs, gewonnen durch Extraktion aus der Pflanze Hypericum (Johanniskraut) bis zu einer Konzentration an polycyklischen Dionen (Hypericin) (C₃₀H₁₆O₈) , Pseudohypericin und/oder Salzen davon) von unter 50% zur Herstellung eines Medikaments zur Behandlung einer Krankheit, verursacht durch einen Virus.

3. Verwendung nach Anspruch 1 oder 2, wobei das Medikament neben dem genannten Stoffgemisch einen pharmazeutisch annehmbaren Träger oder ein pharmazeutisch annehmbares Verdünnungsmittel enthält.

4. Verwendung nach einem oder mehreren der vorhergehenden Ansprüche, wobei das Medikament in einer parenteralen Darreichungsform, in einer festen rektalen Darreichungsform, insbesondere als Suppositorium oder in einer festen oralen Darreichungsform, insbesondere einer Tablette, einem Dragee oder einer Kapsel, mit oder ohne magensaftresistentem Überzug, vorliegt.

5. Verwendung nach Anspruch 4, wobei das Medikament einen Gehalt an polycyklischen Dionen (insbesondere Hypericin) von 1 mg bis 20 mg je parenteraler Dosis, Tablette, Dragee, Kapsel bzw. Suppositorium aufweist.

6. Verwendung nach einem oder mehreren der vorhergehenden Ansprüche, wobei das Virus ein Retrovirus, insbesondere ein humanes Immunodefizienz-Virus (HIV) ist.

7. Verwendung nach einem oder mehreren der vorhergehenden Ansprüche, wobei die Tagesdosis an polycyklischen Dionen (Hypericin (C₃₀H₁₆O₈) , Pseudohypericin und/oder Salzen davon) zwischen 0,01 mg und 10 mg pro Kilogramm Körpergewicht eines zu behandelnden Säugetieres (z.B. eines Menschen) umfasst.

8. Verwendung nach einem oder mehreren der vorhergehenden Ansprüche, wobei das Medikament in Kombination mit einem anderen Medikament verabreicht wird, welches ein oder mehrere Nucleosidanaloga, z.B. 3'-Azido-3'-Deoxythymidin (AZT), enthält.

## Claims

1. Use of a mixture of substances, consisting of one or more polycyclic diones, selected from hypericin (C₃₀H₁₆O₈) , pseudohypericin and salts thereof, in a concentration of between 0.05 % and 50 %, as well as of one or more further constituents occurring in the plant Hypericum (Saint John's wort) in a concentration of between 50 % and 99.95 % for the preparation of a medicament for the treatment of an illness caused by a virus.

2. Use of a mixture of substances, obtained by extraction from the plant Hypericum (Saint John's wort) up to a concentration of polycyclic diones (hypericin) (C₃₀H₁₆O₈), pseudohypericin and/or salts thereof) of less than 50 % for the preparation of a medicament for the treatment of an illness caused by a virus.

3. Use according to Claim 1 or 2, wherein the medicament contains a pharmaceutically acceptable excipient or a pharmaceutically acceptable diluent in addition to the said mixture of substances.

4. Use according to one or more of the preceding claims, wherein the medicament is in a form for parenteral administration, in a solid form for rectal administration, in particular in the form of a suppository, or in a solid form for oral administration, in particular in the form of a tablet, a coated tablet or a capsule, with or without a coating resistant to gastric juices.

5. Use according to Claim 4, wherein the medicament has a polycyclic dione (in particular hypericin) content of 1 mg to 20 mg per parenteral dose, tablet, coated tablet, capsule or suppository.

6. Use according to one or more of the preceding claims, wherein the virus is a retrovirus, in particular a human immunodeficiency virus (HIV).

7. Use according to one or more of the preceding claims, wherein the daily dose of polycyclic diones (hypericin (C₃₀H₁₆O₈), pseudohypericin and/or salts thereof) is between 0.01 mg and 10 mg per kilogram body weight of a mammal (for example a person) to be treated.

8. Use according to one or more of the preceding claims, wherein the medicament is administered in combination with another medicament which contains one or more nucleoside analogues, for example 3'-azido-3'-deoxythymidine (AZT).

## Revendications

1. Utilisation d'un mélange de substances, constitué d'un ou de plusieurs dions polycycliques choisis d'hypericine (C₃₀H₁₆O₈), de pseudo-hypericine et de sels de ces derniers, dans des concentrations comprises entre 0,05 % et 50 %, ainsi que d'un ou de plusieurs autres constituants présents dans la plante appelée hypericum (millepertuis) dans des concentrations comprises entre 50 et 99,95 % pour la fabrication d'un médicament destiné au traitement d'une maladie causée par un virus.

2. Utilisation d'un mélange de substances obtenu par extraction d'hypericum (millepertuis) jusqu'à une concentration de dions polycycliques (hypericine) (C₃₀H₁₆O₈), de pseudohypericine et/ou de sels de ces derniers) inférieure à 50% pour la fabrication d'un médicament destiné au traitement d'une maladie causée par un virus.

3. Utilisation suivant la revendication 1 ou 2, le médicament contenant, outre le mélange de substances mentionné, un support ou un diluant acceptables selon des points de vue pharmaceutiques.

4. Utilisation suivant une ou plusieurs des revendications précédentes, le médicament étant présent sous un mode d'administration parentérale, sous un mode d'administration solide rectale, en particulier sous forme de suppositoire ou sous un mode d'administration solide orale, en particulier sous forme de cachet, de dragée ou de gélule avec ou sans enrobage gastro-résistant.

5. Utilisation suivant la revendication 4, le médicament présentant une teneur en dions polycycliques (en particulier d'hypericine) comprise entre 1 mg et 20 mg par dose parentérale, cachet, dragée, gélule ou suppositoire.

6. Utilisation suivant une ou plusieurs des revendications précédentes, le virus étant un rétrovirus, en particulier un virus de déficience immunitaire humaine (HIV).

7. Utilisation suivant une ou plusieurs des revendications précédentes, la dose journalière de dions polycycliques (hypericine (C₃₀H₁₆O₈), de pseudo-hypericine et/ou de sels de ce dernier) comprenant entre 0,01 mg et 10 mg par kilogramme de poids du corps d'un mammifère à traiter (par exemple d'un être humain).

8. Utilisation suivant une ou plusieurs des revendications précédentes, le médicament étant administré en combinaison avec un autre médicament qui contient un ou plusieurs analogues de nucléosides, par exemple 3'-azido-3'-deoxythymidine (AZT).
